# EUROPEAN PATENT SPECIFICATION

(11) **EP 1 565 173 B1**
(45) Date of publication and mention of the grant of the patent: **14.01.2009**
(21) Application number: 03796465.7
(22) Date of filing: 25.11.2003
(51) Int. Cl.: A61K 31/165, A61K 31/245, A61K 31/445, A61P 25/02, A61P 23/00

(54) **COMPOSITION AND ITS USE FOR TREATING NEUROPATHIC SENSORY LOSS**
ZUSAMMENSETZUNG UND IHRE VERWENDUNG ZUR BEHANDLUNG VON NEUROPATHISCHEM VERLUST DER SENSIBILITÄT
COMPOSITION ET SON UTILISATION POUR LE TRAITEMENT DE LA PERTE SENSORIELLE NEUROPATHIQUE

(30) Priority: 26.11.2002 US 429208 P
(43) Date of publication of application: 24.08.2005
(73) Proprietor: Endo Pharmaceuticals Inc., Chadds Ford, PA 19317 (US)
(72) Inventor: GALER, Bradley, Stuart, West Chester, PA 19382 (US); GAMMAITONI, Arnold, R., Downingtown, PA 19335 (US); DWORKIN, Robert, D., Pittsford, NY 14534 (US)
(74) Representative: Buckley, Guy Julian
(86) International application number: PCT/US2003/037815
(87) International publication number: WO 2004/047819

(56) References cited:
- ESCOBAR P L ET AL: "Teres minor. Source of symptoms resembling ulnar neuropathy or C8 radiculopathy." AMERICAN JOURNAL OF PHYSICAL MEDICINE & REHABILITATION / ASSOCIATION OF ACADEMIC PHYSIATRISTS. UNITED STATES JUN 1988, vol. 67, no. 3, June 1988 (1988-06), pages 120-122, XP008030617 ISSN: 0894-9115
- CALISSI P T ET AL: "PERIPHERAL DIABETIC NEUROPATHY: CURRENT CONCEPTS IN TREATMENT" ANNALS OF PHARMACOTHERAPY, XX, XX, vol. 29, no. 7/8, 1995, pages 769-777, XP000909350 ISSN: 1060-0280
- PATENT ABSTRACTS OF JAPAN vol. 008, no. 205 (C-243), 19 September 1984 (1984-09-19) & JP 59 093018 A (SUMIMOTO KAGAKU KOGYO KK), 29 May 1984 (1984-05-29)
- DEVERS A ET AL: "Topical lidocaine patch relieves a variety of neuropathic pain conditions: an open-label study." THE CLINICAL JOURNAL OF PAIN. UNITED STATES SEP 2000, vol. 16, no. 3, September 2000 (2000-09), pages 205-208, XP008030616 ISSN: 0749-8047

## Description

### FIELD OF THE INVENTION

The present invention relates to decreasing the effects of neuropathically induced negative sensory phenomena (NSP).

### BACKGROUND OF THE RELATED ART

Patients with damaged or dysfunctional peripheral nerves, a condition commonly known as "neuropathy," often develop symptoms of sensory deficits or loss (numbness), such as a decreased ability to feel light touch, pain, proprioception, vibration, warmth or heat, and cool or cold conditions in the area of the nerve damage. Patients may also describe "feelings of numbness" over the affected body region. Such symptoms are conveniently labeled "negative sensory phenomena" or "NSP." NSP are distinguished from Positive Sensory Phenomena (PSP) that are indicated by increased sensitivity, dysesthesia (tingling, pins and needles, etc.), and pain. Some neuropathy patients may experience both NSP and PSP, while others experience only one or the other.

U.S. Patent No. 5,976,547--Archer et al. discloses a flexible wrap of an analgesic and an antiphlogistic including extracts of *arnica montana.* The wrap is used for treating peripheral and central pain, including lower extremity paresthesias, numbness and hyperesthesia associated with diabetic peripheral neuropathy. In addition to the extract of *arnica montana*, the wrap may contain one or more of several therapeutic or pharmaceutical agents including, *inter alia,* lidocaine. Lidocaine is not used to treat numbness.

U.S. Patent No. 6,337,423-Axt et al., discloses the use of local anesthetics including lidocaine for treating neuropathic pain.

U.S. Patent No. 6,147,102--Borgman, discloses the use of clonidine-containing preparations in treating sympathetically maintained peripheral neuropathy.

"Topical lidocaine patch relieves a variety of neuropathic pain conditions : An open-label study", Devers and Galer, Clinical Journal of Pain, 16: 205-208, 2000, discloses the use of lidocaine in a topical patch for the treatment of peripheral neuropathic pain conditions other than postherpetic neuralgia.

Published U.S. Patent Application US2002/0037926, published on March 28, 2002, discloses the use of sodium channel blockers in combination with gabapentin or pregabalin for treating chronic pain or convulsions.

Thus, although local anesthetics for treating neuropathic pain a nd associated PSP (tingling, pins and needles, and pain) are known, none relate to the treatment of neuropathically induced NSP (numbness, decreased sensation). In particular, the treatment of PSP or increased sensitivity, along with the pain, by administering local anesthetics is known. None of the references, however, suggest that a pain reducing treatment would also be applicable for increasing sensation where it is diminished or reducing the sensation of numbness in the region(s) of neuropathy. Therefore, there exists a long-felt and unmet need for methods for treating negative sensory phenomena (NSP).

### SUMMARY OF THE INVENTION

The present invention is as set out in the accompanying claims.

Accordingly, it has now been found that NSP in a neuropathy patient are alleviated by the transdermal administration of a pharmaceutical compound that is applied to the affected area.

The present invention therefore provides the use of certain benzoic acid derivatives in the manufacture of a medicament for treating a sensory loss due to neuropathic NSP by applying an NSP-relieving amount of a pharmaceutical compound to a patient suffering from neuropathic NSP at a location near the sensory loss. The pharmaceutical compound is at least one compound selected from benzoic acid-based anesthetics, specifically benzocaine, procaine, lidocaine, prilocaine, or pharmaceutically a cceptable salts a nd derivatives thereof. In those embodiments
where lidocaine is applied, it is most preferable to utilize a lidocaine patch including a carrier containing 5% lidocaine.

In certain embodiments, the present invention includes the use of the benzoic acid derivatives in the manufacture of a medicament for treating sensory loss due to neuropathic NSP, by transdermal administration of a local anesthetic. This involves applying a composition comprising from about 2 to about 10% by weight of the anesthetic (such as lidocaine) in a form capable of transdermal transport. The lidocaine is absorbed transdermally to provide relief at the site of the neuropathy. Preferably, if a patch is used, the active ingredient is covered with a cover selected from the group consisting of polyvinyl chloride, polyvinylidene chloride, polyethylene, synthetic rubber, woven polyester fabric, and non-woven polyester fabric. In another preferred embodiment, sensory loss due to neuropathic NSP is treated by transdermal administration and more specifically by applying a patch comprising a physiologically acceptable adhesive including from about 2 to about 10% by weight, and more preferably about 5% by weight, of lidocaine in a formulation that provides transdermal transport of the lidocaine, and a non-woven polyester covering. The medicated patch is applied directly to the skin where the patient describes the NSP. The medicated patch does not produce clinically meaningful blood plasma levels of active ingredient.

### DESCRIPTION OF THE PREFERRED EMBODIMENTS

The present invention relates to reducing the effects of neuropathically induced negative sensory phenomena (NSP) such as numbness and decreased sensation. As described above, NSP is manifested as the decreased ability to feel light touch, pain, proprioception, vibration, warmth/heat, and coolness/cold. As described above, NSP may be manifest solely by patient complaint or description of "numbness" in the affected region without the ability to document abnormalities in nerves with electromyography, nerve conduction velocity, or quantitative sensory testing laboratory assessments. Therefore, as used herein, the terms "NSP" or "neuropathic NSP" should be interpreted broadly to include all such neuropathic conditions and indications whether now known or later discovered. Such NSP are, by definition, functional disturbances considered to be caused by neuropathy, unless a temporary external agent is acting, such as an injected temporary anesthetic.

In the present invention, a local anesthetic is used in a medicament which is applied to alleviate neuropathic sensory loss. The anesthetic improves sensation at the site of the application. The anesthetic alleviates the complaint of NSP as described by the patient as numbness. The anesthetic is a benzoic acid derivative, normally used as a local anesthetic, as distinguished from a general anesthetic. Specifically, benzoic acids such as benzocaine and cocaine, meta-aminobenzoic acids such as proparacaine, para-aminobenzoic acids such as procaine, chloroprocaine and tetracaine, and amide-derivatives of benzoic acid such as lidocaine, mepivacaine, bupivacaine and etidocaine are useful in the present invention. Of these, para-aminobenzoic acid derivatives and other amide-derivatives are preferred. More preferred are amide-derivatives. Specifically, it is most preferred if the local anesthetic is lidocaine, and specifically, a patch containing about 5% lidocaine. Lidocaine is a synthetic amide, 2-(diethylamino)-N-2,6-dimethylphenyl)-acetamide (C₁₄H₂₂N₂O) used chiefly in the form of its hydrochloride as a local anesthetic and antiarrhythmic agent. The anesthetic is preferably applied to a patient suffering from NSP at or near, the locus of, the reduced sensation. The locus of the reduced sensation is the spot on the skin of a patient where the reduced sensation is most noticeable, where it is most uncomfortable, or where an identified neuropathy exists. These places usually coincide, but if not, the anesthetic should be applied at one or more of these locations until relief from symptoms is realized. The anesthetic actually increases sensation and improves comfort in NSP patients.

Methods for treating sensory loss due to neuropathic NSP by applying an anesthetic to a patient suffering from neuropathy induced NSP at a location near the sensory loss, as disclosed herein, can utilize an active ingredient selected from benzocaine, procaine, tetracaine, chloroprocaine, propoxycaine, cocaine, proparacaine, mepivacaine, bupivacaine, phenocaine, dibucaine, etidocaine, lidocaine, prilocaine, or pharmaceutically acceptable salts thereof, or alternatively a derivative of one of these active ingredients such as procaine butyrate, procaine borate, etc. Both the anesthetics and derivatives can be used alone or in combination. Those of skill in the art will be able to determine the amounts and concentrations of these active ingredients without undue experimentation in order to create dosages that can be administered transdermally in a manner that is both safe and efficacious. For example, in those embodiments where lidocaine is applied, it is most preferable to utilize a carrier containing about 5% lidocaine, preferably in a patch.

In certain embodiments, the medicament for treating sensory loss due to neuropathic NSP by transdermal administration comprises from 2 to 10%, preferably from 3 to 7% by weight of lidocaine in a form capable of transdermal transport, so that the lidocaine is transported transdermally to provide for relief at the site of the NSP. Preferably, the active ingredient is covered with a material selected from the group consisting of polyvinyl chloride, polyvinylidene chloride, polyethylene, synthetic rubber, woven polyester fabric, and non-woven polyester fabric, to cover and protect the area.

Thus it will be understood that the present invention encompasses use in transdermal patches, which are familiar drug delivery mechanisms to those skilled in the art. As an example, a treatment for sensory loss due to neuropathic NSP using a patch involves administration by applying a physiologically acceptable adhesive including from about 2 to about 10% by weight, and more preferably about 5% by weight, of lidocaine. The lidocaine is contained in a formulation that provides transdermal transport of the lidocaine from the adhesive. The patch includes a non-woven polyester covering.

The present invention is also directed to the use of the benzoic acid derivatives in the preparation of a composition for treating sensory loss due to neuropathic NSP by transdermal administration, wherein the composition comprises a plaster or gel containing from 2 to 10% by weight of lidocaine. Preferably, the composition contains lidocaine in an amount of 5% by weight, and is combined with a cover selected from the group consisting of polyvinyl chloride, polyvinylidene chloride, polyethylene, synthetic rubber, woven polyester fabric, and non-woven polyester fabric. In preferred embodiments, the formulation provides at least eight hours of relief from NSP.

Where the medicament comprises a plaster for treating sensory loss, the plaster contains a physiologically acceptable adhesive, comprising from about 2 to about 10% by weight of lidocaine, most preferably about 5% by weight, and a non-woven polyester covering. Certain preferred embodiments also provide a gel comprising from about 2 to about 10% by weight of lidocaine, most preferably about 5%, wherein the formulation comprises about 70 to about 90% weight of an anhydrous vehicle, such as ethanol, isopropanol, propylene glycol, or glycerin, along with about 0.1 to about 5% weight of a physiologically acceptable gelling agent, about 2 to about 20% weight of a nonionic surfactant, and up to about 10% weight of physiologically acceptable excipients.

It has previously been assumed that positive sensory phenomena ("PSP") is caused by an increase in spontaneous nerve discharge from damaged and dysfunctional peripheral nerves. Therefore, it was expected that transdermally administered sodium channel blockers, including local anesthetics such as lidocaine, could reduce the increased spontaneous discharges responsible for PSP and hence result in relief of pain and dysesthesia. Based on the current understanding of the underlying etiology of NSP it would be unexpected that an anesthetic would effectively mitigate the negative effects of NSP. It has now been found, however, that the application of local anesthetics such as lidocaine can effectively treat NSP. For example, some diabetic neuropathy patients reported both pain relief and improved NSP, that is, the patients experienced pain relief, improvement of sensory loss (decreased numbness), and improved tactile response (they could better feel objects touching their skin). Thus the anesthetic as applied in this embodiment decreased numbness. This is contrary to all prior teachings about anesthetics.

It is believed that the mechanism of action of the present invention is that NSP may be caused, at least in some patients, by increased spontaneous discharges in a special population of peripheral nerves whose function is to relay perceptive information of "numbness" and other NSP. Therefore, in a manner similar to treating PSP, transdermally administered anesthetics, in appropriate concentrations, reduce the ectopic discharges in these dysfunctional NSP-responsible peripheral nerves. Of course, the present invention is not intended to be limited by this theory.

## Claims

1. The use of a benzoic acid derivative selected from the group consisting of benzocaine, procaine, tetracaine, chloroprocaine, propoxycaine, cocaine, proparacaine, mepivacaine, bupivacaine, phenocaine, dibucaine, etidocaine, lidocaine, prilocaine, and pharmaceutically acceptable salts thereof, in the manufacture of a medicament for the treatment of neuropathic negative sensory phenomena at the locus of the negative sensory phenomena.

2. The use of claim 1, wherein the benzoic acid derivative is lidocaine.

3. The use as claimed in any one of claims 1 to 2, wherein the medicament is contained in a patch.

4. The use of claim 3, wherein said patch contains between 2% and 10% lidocaine.

5. The use of claim 4, wherein said patch contains 5% lidocaine.

6. The use according to claim 3, wherein the patch further comprises a cover over said medicament, and wherein said cover is formed from a material selected from the group consisting of polyvinyl chloride, polyvinylidene chloride, polyethylene, synthetic rubber, woven polyester fabric, and non-woven polyester fabric.

7. The use of claim 1, wherein the medicament for treating neuropathic negative sensory phenomena is intended for treatment by transdermal administration of lidocaine, and wherein the medicament comprises 2 to 10 % by weight lidocaine contained in a non-woven polyester cloth including a physiologically acceptable adhesive.

8. The use according to claim 7, wherein said lidocaine is present in 5% by weight.

9. Use as claimed in any one of claims 1 to 8 for the manufacture of a medicament for decreasing numbness of the skin by applying the medicament at the locus of a site of said numbness.

## Patentansprüche

1. Die Verwendung eines Benzoesäurederivats, ausgewählt aus der Gruppe bestehend aus Benzocain, Procain, Tetracain, Chlorprocain, Propoxycain, Cocain, Proparacain, Mepivacain, Bupivacain, Phenocain, Dibucain, Etidocain, Lidocain, Prilocain und pharmazeutisch verträglichen Salzen davon, bei der Herstellung eines Medikaments zur Behandlung von neuropatischen negativen sensorischen Phänomenen an der Lokalisation der negativen sensorischen Phänomene.

2. Die Verwendung nach Anspruch 1, wobei das Benzoesäurederivat Lidocain ist.

3. Die Verwendung wie in einem der Ansprüche 1 bis 2 beansprucht, wobei das Medikament in einem Pflaster enthalten ist.

4. Die Verwendung nach Anspruch 3, wobei das Pflaster zwischen 2 % und 10 % Lidocain enthält.

5. Die Verwendung nach Anspruch 4, wobei das Pflaster 5 % Lidocain enthält.

6. Die Verwendung nach Anspruch 3, wobei das Pflaster ferner eine Abdeckung über dem Medikament umfasst und wobei die Abdeckung aus einem Material hergestellt ist, ausgewählt aus der Gruppe bestehend aus Polyvinylchlorid, Polyvinylidenchlorid, Polyethylen, synthetischer Gummi, gewobener Polyesterstoff und nicht gewobener Polyesterstoff.

7. Die Verwendung nach Anspruch 1, wobei das Medikament zur Behandlung von neuropatischen negativen sensorischen Phänomenen zur Behandlung durch transdermale Verabreichung von Lidocain vorgesehen ist und wobei das Medikament 2 bis 10 Gew.-% Lidocain umfasst, das in einem nicht gewobenen Polyesterstoff enthalten ist, der ein physiologisch verträgliches Adhäsiv einschließt.

8. Die Verwendung nach Anspruch 7, wobei das Lidocain zu 5 Gew.-% vorhanden ist.

9. Verwendung wie in einem der Ansprüche 1 bis 8 beansprucht für die Herstellung eines Medikaments zur Verringerung von Gefühllosigkeit der Haut durch Anwenden des Medikaments an der Lokalisation einer Stelle der Gefühllosigkeit.

## Revendications

1. Utilisation d'un dérivé de l'acide benzoïque sélectionné dans le groupe constitué de benzocaïne, procaïne, tétracaïne, chloroprocaïne, propoxycaïne, cocaïne, proparacaïne, mépivacaïne, bupivacaïne, phénocaïne, dibucaïne, étidocaïne, lidocaïne, prilocaïne et de leurs sels acceptables au plan pharmacologique, dans la fabrication d'un médicament destiné au traitement de phénomènes sensoriels négatifs névropathiques sur le lieu des phénomènes sensitifs négatifs.

2. Utilisation selon la revendication 1, où le dérivé de l'acide benzoïque est la lidocaïne.

3. Utilisation selon l'une quelconque des revendications 1 à 2, où le médicament est contenu dans un patch.

4. Utilisation selon la revendication 3, où ledit patch contient entre 2 % et 10 % de lidocaïne.

5. Utilisation selon la revendication 4, où ledit patch contient 5 % de lidocaïne.

6. Utilisation selon la revendication 3, où le patch contient également un couvercle sur ledit médicament, et où ledit couvercle est formé d'un matériau sélectionné dans le groupe constitué de polychlorure de vinyle, polychlorure de vinylidène, polyéthylène, caoutchouc synthétique, étoffe de polyester tissé et étoffe de polyester non tissé.

7. Utilisation selon la revendication 1, où le médicament destiné au traitement de phénomènes sensoriels négatifs névropathiques est prévu pour un traitement par administration transdermique de lidocaïne, et où le médicament comprend 2 à 10 % en poids de lidocaïne contenus dans une toile de polyester non tissé comprenant un adhésif physiologiquement acceptable.

8. Utilisation selon la revendication 7, où ladite lidocaïne est présente à raison de 5 % en poids.

9. Utilisation selon l'une quelconque des revendications 1 à 8, pour la fabrication d'un médicament destiné à réduire l'engourdissement de la peau par l'application du médicament à l'emplacement d'un site dudit engourdissement.
